Europäisches Patentamt

(19) European Patent Office

Office européen des brevets

(11) **EP 0 900 560 A1**

(12) **EUROPEAN PATENT APPLICATION**

(43) Date of publication:
**10.03.1999 Bulletin 1999/10**

(51) Int. Cl.⁶: **A61K 7/16**, C09D 193/02

(21) Application number: **98117005.3**

(22) Date of filing: **08.09.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **09.09.1997 JP 285951/97**
**22.10.1997 JP 309268/97**
**23.02.1998 JP 58871/98**

(71) Applicant: **Oka, Hironori**
**Okayama-shi, Okayama 703 (JP)**

(72) Inventor: **Oka, Hironori**
**Okayama-shi, Okayama 703 (JP)**

(74) Representative:
**Patentanwälte**
**Dr. Solf & Zapf**
**Candidplatz 15**
**81543 München (DE)**

(54) **Tooth coating composite and its preparation**

(57)    The present invention, having the features mentioned above, can perform the following functions. The composite of the present invention comprising shellac dissolved in alcohol and at least one of antibacterial constituent, anti-bacteria antibody, and efficacious constituent is applied to a tooth surface to form an antibacterial film on the tooth surface such that it can prevent effectively dental caries and periodontal disease and cure periodontal disease. Further, it is possible to apply the composite to a tooth without any special technical skill such that it is quite easy to prevent dental caries and periodontal disease without any help of the dentist.

## Description

### Field of the Invention

The present invention relates to a tooth coating composite, more particularly, to a tooth coating composite applying on a tooth to enhance a preventive effect against dental caries and periodontal disease and its preparation.

### Background of the Invention

It is very important to prevent tooth pyogenesis (designated "dental caries" thereafter) and periodontal disease in order to keep not only good intra-oral health but also good health of the whole body. In order to prevent dental caries and periodontal disease (designated "the disease" thereafter), a toothbrush cleaning method and other methods are usually used to remove food and other matter stuck to a tooth so as to prevent dental plaque from sticking to the tooth and to keep intra-oral cleanliness. However, it is very difficult to prevent the disease completely by the methods mentioned above. Therefore, other methods for preventing the disease effectively have been investigated. A curing method for periodontal disease has also bee investigated.

For example, fluoride coating on a tooth so as to strengthen resistance to dental caries, and gargling with bactericidal gargle so as to sterilize intra-oral germs and bacteria that may cause the disease, have been applied. However, it is impossible for a layman to apply fluoride coating on the tooth because it requires a high technical skill so that only a dentist can apply it. Gargling with the bactericidal gargle, however, has a sterilizing effect for only a short period. No effective curing method that a layman can apply for periodontal disease has been known yet.

It is a purpose of the present invention to provide a tooth coating composite that can be applied easily by the layman in order to prevent the disease and to cure periodontal disease and that has the preventive effect for a certain period of time.

### Summary of the Invention

The present invention seeks to achieve the purpose by providing a tooth coating composite comprising shellac dissolved in alcohol and its preparation. In accordance with the present invention, the composite is applied to a tooth such that shellac coating may cover a surface of the tooth and can prevent germs and bacteria that may cause the disease from sticking and proliferating. Thus, periodontal disease can be cured effectively. These preventive and therapeutic effects last for a certain period of time that the shellac coating exists. The composite also may include anti-bacterial constituent, anti-bacteria antibody and efficacious constituent without inhibiting each function. Therefore, film or coating made from the composite including anti-bacterial constituent, anti-bacteria antibody and efficacious constituent can have enhanced effects on preventing germs and bacteria that may cause the disease from sticking and proliferating with the help of each function so that periodontal disease can be cured more effectively.

The anti-bacterial constituent comprises at least one of propolis, hinokitiol, chitin chitosasn and disinfectant, the disinfectant further comprising at least one of quaternary ammonium salt or quaternary ammonium salt-type disinfectant, fluoride or fluoride-type disinfectant, bisbiguanide derivative or bisbiguanide derivative-type disinfectant, phenol or phenol-type disinfectant, amphoteric surface active agent or amphoteric surface active agent-type disinfectant. The quaternary ammonium salt or quaternary ammonium salt-type disinfectant further comprises at least one of cetylpyridinium chloride, benzalkonium chloride, benzethonium chloride, and dequalinium chloride. The fluoride or fluoride-type disinfectant further comprises at least one of sodium fluoride, sodium monofluorophosphate, and strontium fluoride. The bisbiguanide derivative or bisbiguanide derivative-type disinfectant further comprises chlorhexidine. The phenol or phenol-type disinfectant further comprises at least one of triclosan, thymol, and isopropyl methyl phenol. The amphoteric surface active agent or amphoteric surface active agent-type disinfectant further comprises alkyldiaminoethylglycine chloride.

The anti-bacteria antibody comprises anti-dental-caries-bacteria and/or anti-periodontal-disease-bacteria antibody. The anti-dental-caries-bacteria antibody further comprises IgY antibody to Streptococcus mutans. The anti-periodontal-disease-bacteria antibody further comprises at least one of IgY antibody to Actinobacillus actinomycetemcomitans, IgY antibody to Porphyronomas gingivalis, IgY antibody to Fusobacterium nucleatum, IgY antibody to Campylobacter rectus, IgY antibody to Bacteroides forsythus, and IgY antibody to Treponema denticola.

The efficacious constituent comprises at least one of tranexamic acid, allantoin, allantoindihydroxyaluminium, allantoinchlorohydroxyaluminium, ε-aminocaproic acid, lysozyme, sodium chloride, azulene, dl-α-tocopherol acetate, dl-α-tocopherol nicotinate, epidihydrocholesterol, dihydrocholesterol, Phellodendron Bark extract, zinc citrate, gantrosan, catechin, β-glycyrrhizinate, glycyrrhetic acid.

Tooth coating composites have been developed for a cosmetic purpose and are on the market now. However, the coatings with the tooth coating composites are thick and have a sticky surface. Moreover, it takes a long time (longer than several tens of seconds) to form (or harden) the coating (or film). Most coatings help dental caries (a dental plaque) to stick to the tooth and do not perform the preventive effect against dental caries well. On the contrary, a coating formed with the tooth coating composite of the present invention is very thin and has little stickiness. It takes only a few second to form the coating (or film). Further, the coating formed on a living tooth with the composite of the present invention has a high resistance to exfoliation by a mechanical rubbing such as mastication, toothbrushing, etc. and by a chemical or physical environment change such as saliva, temperature difference, etc. It also has a high covering effect and a good gloss, does not become cloudy for a long time, and is not toxic to a human body.

The shellac is a chief component of the tooth coating composite of the present invention and is made from secretory fluid of a scale insect. It is insoluble to water and oil, but soluble to alcohol, and is often used in food, cosmetic, and medical area. Therefore, the shellac is usually applied in a form of alcohol solution. The alcohol solution including the shellac forms a thin shellac film since the alcohol constituent is vaporized after applying the solution. Further, if the dental caries (a dental plaque) ejects acid to dissolve the tooth, the shellac film can protect the tooth from the acid because the shellac is slightly soluble to alkali but is almost insoluble to acid.

Referring to a mixing ratio of shellac with alcohol, weight of shellac is preferably form 1% to 50 % of the total weight of shellac and alcohol, more preferably is from 15% to 35 % of the total weight. If the concentration of shellac is too low, the formed film becomes too thin to have enough strength or a good gloss. On the other hand, if the concentration of shellac is too high, it is difficult to apply the solution because the formed film hardens too fast and the formed film also becomes too thick. Any kind of alcohol can be used as a solvent if it dissolves shellac well. However, ethanol is preferable if it is taken into consideration to apply the solution intra-orally.

The composite also may include anti-bacterial constituent, anti-bacteria antibody and efficacious constituent without inhibiting each function. Therefore, the film or coating made from the composite including these can have enhanced anti-bacterial and medicinal functions.

In accordance with the present invention, the propolis, one of the antibacterial components, is made from mixtures that honeybees make with sap and their secretion such as saliva and has a strong antibacterial function such that it is used for a health food and a folk medicine. The hinokitiol is 4-isopropyltropolone and is included in Chamaecyparis taiwanensis material as a red iron complex salt. People pay attention to the hinokitiol because of its properties such as antibacterial function. All of propolis, hinokitiol and chitin chitosan are soluble to alcohol and are readily added to alcohol solution of shellac. If a simple shellac alcohol-solution is applied to form a film, the film becomes cloudy so that a tooth surface with the applied composite may lose a good gloss. However, addition of the propolis and/or the hinokitiol to the shellac alcohol-solution effectively prevents the formed film from becoming cloudy. Thus, the composite including the propolis and/or the hinokitiol of the present invention does not have to have a special ingredient to prevent cloudiness although a specific perfume must be added to prevent cloudiness in the prior art (Japanese patent publication number "Toku Kai Hei" 07-17822).

Further, it was found that the film formed from the shellac alcohol-solution including the hinokitiol and the propolis is thin and little sticky and that it took a few second to form the film. In addition, the shellac itself has an electrostatic repulsive property to the dental caries bacteria and the periodontal disease bacteria (the dental plaque). It was also found that the antibacterial inclusion such as hinokitiol, propolis, etc. synergistically increases the electrostatic repulsive property. Thus, in accordance with the present invention, the film formed from the composite including propolis or hinokitiol repels the dental caries bacteria and the periodontal disease bacteria (the dental plaque) electrostatically such that the electrostatic repulsive property may prevent the dental caries bacteria and the periodontal disease bacteria (the dental plaque) from sticking to the tooth surface and that its antibacterial property can prevent the dental caries bacteria (the dental plaque) from proliferating on the tooth surface (the film surface).

In accordance with the present invention, the disinfectant, one of the antibacterial components, further comprises at least one of quaternary ammonium salt or quaternary ammonium salt-type disinfectant, fluoride or fluoride-type disinfectant, bisbiguanide derivative or bisbiguanide derivative-type disinfectant, phenol or phenol-type disinfectant, and amphoteric surface active agent or amphoteric surface active agent-type disinfectant. Some of these substances may preferably be applied if they have been used or combined in toothpaste and if they have been recognized to have prominent intra-oral disinfection effects. By way of example, cetylpyridinium chloride, benzalkonium chloride, benzethonium chloride, dequalinium chloride, domiphen bromide, and so on may be used as quaternary ammonium salt or quaternary ammonium salt-type disinfectant. As fluoride or fluoride-type disinfectant, sodium fluoride, sodium monofluorophosphate, strontium fluoride and so on may be used. As bisbiguanide derivative or bisbiguanide derivative-type disinfectant, chlorhexidine, arecolidine and so on may be used. As phenol or phenol-type disinfectant, triclosan, thymol, isopropyl methyl phenol and so on may be used. As amphoteric surface active agent or amphoteric surface active agent-type disinfectant, alkyldiaminoethylglycine chloride etc. may be used. Among these disinfectants, cetylpyridinium chloride, benzalkonium chloride, benzethonium chloride, dequalinium chloride, sodium fluoride, sodium monofluorophosphate, strontium fluoride, chlorhexidine, triclosan, thymol, isopropyl methyl phenol, and alkyldiaminoethylglycine chloride are preferable since these are extensively used in toothpaste and are recognized to be safe and to have prominent intra-oral disinfection effects. In accordance with the present invention, the composite may include above-mentioned components and their salts or salt-type materials if the components are acid, base, or acid/base-type. For example, the term "chlorhexidine" may include chlorhexidine gluconate.

In accordance with the present invention, anti-dental-caries antibody, and one of the antibacterial components, may be antibody to any kind of the dental caries bacteria, preferably may be IgY antibody to a typical dental caries, Streptococcus mutans. It is possible to prevent the dental caries from sticking and proliferating on the tooth surface if the composite of the present invention, to which antibacterial antibody is added, is applied to the tooth. The term "IgY antibody" represents antibody obtained from yolk in which descendible immune antibody that a hen formed in its blood is transferred and accumulated. It is called "IgY" because it is maternal antibody that is obtained from the yolk. Explaining a preparation method of the IgY antibody briefly, firstly an egg-laying hen is immunized against an ingested antigen and lays an egg having antibody in yolk. Secondly the yolk of the egg is carageenan-treated, where the carageenan treatment agglutinates yolk lipoprotein such that it may separate yolk water-soluble protein having IgY antibody from the yolk lipoprotein. Thirdly, the carageenan-treated yolk is centrifuged. Thus, the yolk lipoprotein is removed as a precipitate and the supernatant liquid is collected. Fourthly, since the supernatant liquid has various kinds of water-soluble protein, it is fractionated (ion chromatography), salted out, and processed further to obtain desired IgY antibody. The IgY antibody is, for example, referred to "H. Hatta, M. Kim, and T. Yamamoto, Agric. Biol. Chem., 54, 2531(1990)".

The anti-periodontal-disease-bacteria antibody may be antibody to any kinds of periodontal disease bacteria, more preferably may be antibody to typical periodontal disease bacteria as shown below: IgY antibody to Actinobacillus actinomycetemcomitans, IgY antibody to Porphyronomas gingivalis, IgY antibody to Fusobacterium nucleatum, IgY antibody to Campylobacter rectus, IgY antibody to Bacteroides forsythus, IgY antibody to Treponema denticola and so on.

It is possible to prevent the periodontal disease bacteria from sticking and proliferating on the tooth surface if the composite of the present invention, to which anti-periodontal-disease-bacteria antibody is added, is applied to the tooth. Although various manufacturing methods of preventive vaccines against the periodontal disease bacteria are disclosed (Japanese patent publication number "Toku Kai Sho" 59-128338, "Toku Kai Sho" 61-140527, "Toku Kai Hei" 05-132428, and "Toku Kai Hei" 08-176014), it is preferable to use a safe vaccine to human body.

In a method for preparing the composite of the present invention including anti-dental-caries-bacteria antibody and/or anti-periodontal-disease-bacteria antibody, the antibodies lose their activity if the powdered antibodies are dissolved to alcohol directly. In order to prevent the activity loss, the composite is prepared in a method comprising the following steps:

making a mixture of anti-dental-caries-bacteria antibody and/or anti-periodontal-disease-bacteria antibody, saccharide derivative, and water; and
mixing the mixture with shellac dissolved in alcohol. Any kind of saccharide derivative, e.g., xylitol, palatinose, maltitol, isomaltose oligosaccharide and trehalose may be used in the method.

The composite of the present invention may comprise the propolis and/or the hinokitiol, and the anti-dental-caries-bacteria antibody and/or the anti-periodontal-disease-bacteria antibody. The composite including these types of materials has more preventive property against the disease than the composite including only the propolis and/or the hinokitiol, or only the anti-dental-caries-bacteria antibody and/or the anti-periodontal-disease-bacteria antibody. In particular, it is preferable to apply the composite including the propolis and the anti-dental-caries-bacteria antibody and/or the anti-periodontal-disease-bacteria antibody because a synergistic effect of these antibodies can be utilized.

In accordance with the present invention, tranexamic acid, allantoin, allantoindihydroxyaluminium, allantoinchlorohydroxyaluminium, $\varepsilon$-aminocaproic acid, lysozyme, sodium chloride, azulene, dl-$\alpha$-tocopherol acetate, dl-$\alpha$-tocopherol nicotinate, epidihydrocholesterol, dihydrocholesterol, Phellodendron Bark extract, zinc citrate, gantrosan, catechin, $\beta$-glycyrrhizinate, glycyrrhetic acid that may added to the composite as efficacious constituent are in common use for toothpaste preventive against periodontal disease. They are recognized as alleviating effective component and their safe and pharmacological functions are well known.

Tranexamic acid is known to have anti-inflammation, anti-plasmin and anti-allergy functions. Allantoin, allantoindihydroxyaluminium, and allantoinchlorohydroxyaluminium are known to have cell proliferation (granulation), removal of necrotized tissue, anti-allergy, leucocyte stimulation, and hemostasis functions. Epsilon($\varepsilon$)-aminocaproic acid is known to have an anti-plasmin function. Lysozyme is known to have anti-inflammation, tissue repair, prevention of bacteria invasion, and hemostasis functions. Sodium chloride is known to have blood circulation facilitation, astringency (tissue fluid exchange), and edema suppression functions. Azulene is known to have anti-inflammation, anti-allergy (antihistamine), and edema suppression functions. Dl-$\alpha$-tocopherol acetate and dl-$\alpha$-tocopherol are known to have peripheral blood circulation facilitation, biomembrane stabilization (blood vessel reinforcement), in vivo anti-oxidation, platelet aggregation prevention, and blood vessel permeating prevention functions. Epidihydrocholesterol and dihydrocholesterol is known to have anti-inflammation, saliva cleanup facilitation, and oxygen activation functions.

Phellodendron Bark extract is known to have anti-inflammation, edema suppression, erythrocyte membrane stabilization, and astringency functions. Zinc citrate and gantrosan are known to have a function to prevent dental plaque adhesion. Catechin is known to have a function to prevent bad breath. Beta($\beta$)-glycyrrhizinate and glycyrrhetic acid are known to have anti-inflammation, anti-allergy, and hemolysis prevention functions. Thus, the composite may include appropriate efficacious components mentioned above for required efficacy of formed coating (film).

Any amount of antibacterial constituent, anti-bacteria antibody, and efficacious constituent may be added to the composite of the present invention. The amount of each component may freely be adjusted to required effects of the component. However, coating (film) formed on a tooth from the composite including any of these components may not have enough effects if the concentration of the components is too low. On the contrary, if the concentration of the components is too high, the coating may not have enough strength. Therefore, the concentration of the components may preferably be adjusted within a range satisfactory for both requirements.

Anti-bacterial constituent, anti-bacteria antibody, or efficacious constituent other than mentioned above may be added to the composite of the present invention. For example, alcohol-soluble chitosan may be added.

The composite of the present invention may have one or more materials selected from brightener, pigment, and perfume if necessary.

In order to give a good appearance of a tooth with a good gloss on a formed film, the composite of the present invention may have brightener. Any kind of the brightener may be used if it is stable in the composite. It is preferable that one or more brighteners are selected from fish scale flake, titanated mica, sericite, muscovite, Pinctada shell flake, Abalone shell flake, and Rochina niloticus shell flake since the brightener should have no toxin and have a pearl gloss close to a natural tooth.

In order to give a good appearance of a tooth with a color tone on a formed film, the composite of the present invention may have pigment. Any kind of the pigment may be used if it is stable in the composite. It is preferable that one or more pigments are selected from titanium oxide, titanated mica, natural pearl, yellow oxide of iron, calcium (II) phosphate, calcium carbonate, hydroxyapatite, and legally permitted dye since the pigment should not have any toxin. The legally permitted dye comprises caramel, Green 3, Blue 1, Yellow 4, Yellow 203, and Red 106. The legally permitted dye further comprises Amaranth, Erythrosine, New coccin, Phloxine B, Rose bengal, Acid red, Tartrazine, Sunset yellow FCF, Fast green FCF, Briliant blue FCF, and Indigo Carmine. These are names and code numbers permitted by Japanese government and confirmed to be safe for intra-oral use. It is especially preferable to add a proper quantity of titanium oxide to the composite since it gives white color tone on the tooth surface.

Since anti-bacterial constituent, anti-bacteria antibody, or efficacious constituent dissolved in alcohol may has a peculiar odor, it is preferable to add perfume to the composite of the present invention. Any kind of perfume may be used if it is alcohol-soluble. As mentioned above, it is disclosed that addition of a specific perfume to shellac alcohol-solution prevents a formed and hardened film from becoming cloudy (Japanese patent publication number "Toku Kai Hei" 07-17822). However, the composite of the present invention, if it includes propolis and/or hinokitiol, has enough preventive effect to cloudiness. For example, any of ethyl acetate, amyl formate, lavender oil, rosemary oil, peppermint and other mints, wintergreen, cassia, eugenol, saccharin sodium, menthol and other natural extract, methyl butyrate, isoamyl isovalerate, benzyl acetate, and isoamyl acetate may be used preferably.

**Brief Description of the Drawings**

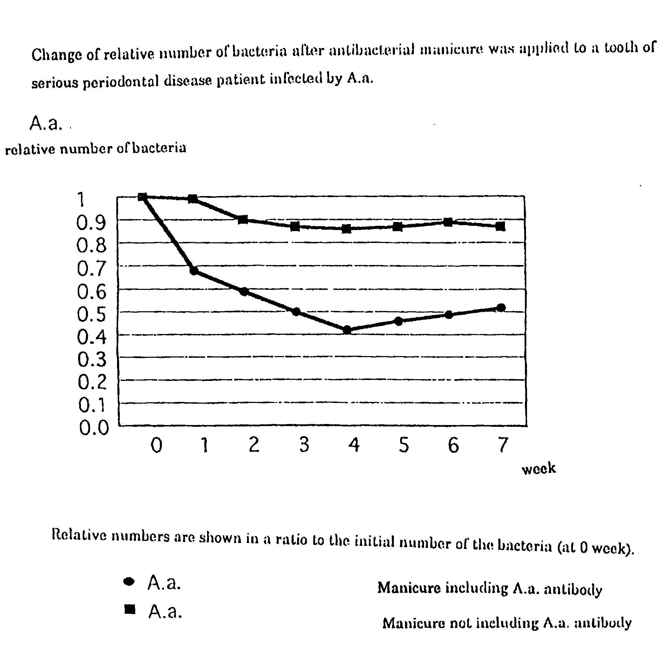

FIG. 1 shows changes of relative number of bacteria when the composite having an anti-periodontal-disease-bacteria antibody (antibody to Actinobacillus actinomycetemcomitans) was applied to a tooth. From the graph, an effect of the composite of the present invention is seen.

**Detailed Description of the Invention**

While a preferred embodiment of the present invention is described in detail thereafter, this is not done for purposes of limitation but by way of illustration.

**Example 1**

A powder of 27.0 g of dried transparent white shellac was dissolved in 56.0 g of 99.5 % absolute ethanol. To the solution, 2.0 g of propolis was added and the mixture was stirred to be homogenized. To the mixture, 2.0 g of fish scale flake paste (25 wt% of fish scale flake kneaded with 75 wt% of 99.5 % absolute alcohol) was added and the mixture was stirred with a mixer. The tooth coating composite obtained through these processes had a light brownish tint and was a suspension of high flowability. When the suspension was applied by brush to a living tooth, it was dried out in three second to form a film. The film had a very similar tint and pearl gloss to those of the tooth enamel. The film had a high resistance to exfoliation and it was not exfoliated for several days with mastication of regular eating. However, some may feel uncomfortable since the shellac and the propolis have a peculiar odor. Thus, alcohol-soluble perfume may be mixed if necessary.

## Example 2

The following materials were mixed in a similar manner to that of the example 1.

| Shellac | 27.0 g |
|---|---|
| Absolute ethanol | 56.0 g |
| Hinokitiol | 2.0 g |
| Amyl formate | 7.0 g |
| Lavender oil | 6.0 g |

A peculiar odor of the shellac could be suppressed by the amyl formate and the lavender oil such that the peculiar odor might be not felt when the composite was applied. Addition of the hinokitiol could increase an antibacterial function.

## Example 3

The following materials were mixed in a similar manner to that of the example 1.

| Shellac | 27.0 g |
|---|---|
| Absolute ethanol | 56.0 g |
| Propolis | 2.0 g |
| Hinokitiol | 2.0 g |
| Amyl formate | 7.0 g |
| Lavender oil | 6.0 g |

A peculiar odor of the propolis and the shellac could be suppressed by the amyl formate and the lavender oil such that the peculiar odor might be not felt when the composite was applied. Addition of the propolis and the hinokitiol could increase an antibacterial function very much.

## Example 4

The following materials were mixed in a similar manner to that of the example 1.

| Shellac | 27.0 g |
|---|---|
| Absolute ethanol | 56.0 g |
| Propolis | 2.0 g |
| Hinokitiol | 2.0 g |
| Fish scale flake paste | 2.0 g |
| Peppermint | 4.0 g |

(continued)

| Ethyl acetate | 7.0 g |
|---|---|

Addition of the fish scale flake paste could give the film a pearl gloss. Addition of the peppermint could supply freshness in a buccal cavity.

**Example 5**

The following materials were mixed in a similar manner to that of the example 1.

| Shellac | 27.0 g |
|---|---|
| Absolute ethanol | 56.0 g |
| Propolis | 2.0 g |
| Hinokitiol | 2.0 g |
| Fish scale flake paste | 2.0 g |
| Titanium oxide | 0.4 g |
| Peppermint | 4.0 g |
| Ethyl acetate | 6.5 g |

Addition of the titanium oxide could give the film a white color tone.

**Example 6**

The following materials were mixed in a similar manner to that of the example 1. In this example, shellac (emulsoid white shellac) was used to give a hardened film a white opaque color.

| Shellac (emulsoid white shellac) | 27.0 g |
|---|---|
| Absolute ethanol | 56.0 g |
| Propolis | 2.0 g |
| Hinokitiol | 2.0 g |
| Fish scale flake paste | 2.0 g |
| Titanium oxide | 0.4 g |
| Ethyl acetate | 6.5 g |

Any composites of the examples 1 to 6 were quite stable. And none of them become cloudy even after a long period of storage. None of the films formed from the composites of the examples 1 to 6 became cloudy and every coated tooth had a beautiful surface with a gloss.

Next, antibacterial effects of these films were examined. As a blank reference, a tooth smooth surface was used. A bacteria sticking ratio was measured on a tooth smooth surface where each composite of the examples 1 to 3 was applied to form a film. Each extracted tooth with a film of each composite was immersed in 2 % sucrose water-solution. The bacteria sticking ratios of Streptococcus mutans (designated "S.m." thereafter) and Streptococcus sobrinus (designated "S.s." thereafter) on the tooth smooth surface were measured after a culture at 37° for 48 hours. The results of S.m. are summarized in Table 1 and the results of S.s. are summarized in Table 2.

Table 1

| Bacteria sticking ratio (%) of S.m. on tooth smooth surface treated with the composite | | | |
|---|---|---|---|
| | Bacteria sticking ratio | ± | Standard deviation (%) |
| Blank | 91.2 | ± | 6.7 |
| Example 1 | 35.7 | ± | 9.6 |
| Example 2 | 30.4 | ± | 11.2 |
| Example 3 | 21.3 | ± | 8.1 |

Table 2

| Bacteria sticking ratio (%) of S.s. on the tooth smooth surface treated with the composite | | | |
|---|---|---|---|
| | Bacteria sticking ratio | ± | Standard deviation (%) |
| Blank | 82.6 | ± | 5.4 |
| Example 1 | 27.9 | ± | 14.9 |
| Example 3 | 17.5 | ± | 8.1 |

From Tables 1 and 2, it is understood that the addition of the propolis (Example 1) and the hinokitiol (Example 2) prevented dramatically the bacteria from sticking to the surface. It is also understood that addition of both propolis and hinokitiol (Example 3) could further lower the ratios.

(Preparation of anti-periodontal-disease-bacteria antibody)

Actinobacillus actinomycetemcomitans (designated "A.a." thereafter) Y4 strain was inoculated into Todd-Hewitt broth to which 1 % yeast extract was added, and cultured in an incubator containing 5 % $CO_2$ at 37° for three days. The cultured bacteria was collected with a centrifugal separator, was irrigated three times with physiological saline solution, was suspended with physiological saline solution, was autoclaved at 121° for 15 minutes. After cooling the treated bacteria mixture, it was centrifuged with 10000xg for 20 minutes and the supernatant liquid was extracted. Physiological saline solution was added to the precipitate and the above-mentioned extraction was repeated. The extracted supernatant liquid was collected, was dialyzed well with distilled water, and was freeze-dried. The obtained material was called bacterial pellicle polysaccharide.
The bacterial pellicle polysaccharide of A.a. was dissolved in 0.01 M Tris hydrochloric acid (HCl) buffer solution (pH 8.2) to be 100 mg/ml solution and was dialyzed with the same butter solution for two days. This was anion-exchange-chromatographed in DEAE-SephadexA-25 column (Pharmacia, Uppsala, Sewden) equilibrated with said buffer solution. In other words, it was eluted with said buffer solution and eluted with gradient concentration from 0 to 1 M sodium chloride solution. Saccharine peak fractions including not-adsorbed saccharine in the column were collected and concentrated with a rotary evaporator. The concentrated substance was dialyzed well with distilled water. The dialyzed concentrated substance was gel-filtrated with SephacrylS-300 column (Pharmacia). Thus, simple saccharide peak fraction was collected and freeze-dried. This was called purified polysaccharide antigen.
Porphyronomas gingivalis (designated "P.g." thereafter) strain 381 was cultured for two days in Todd-Hewitt broth to which hemin and menadione were added and the bacteria was collected. It was processed in a similar manner to that for A.a. to obtain bacterial pellicle polysaccharide of P.g. The bacterial pellicle polysaccharide of P.g. was also processed in a similar manner to that for A.a. to obtain purified polysaccharide antigen of P.g. Although the bacterial pellicle polysaccharide of P.g. was fractionated into a plurality of saccharide peaks in anion exchange chromatography, saccharide peak fractions eluted around 0.05 to 0.25 M NaCl were most effective and these peak fractions were collected in the present example.
The obtained purified polysaccharide antigen was dissolved in 1 M sodium carbonate water-solution, mixed with cyanogen bromide, and stirred at 4°C for 6 minutes for activation. The mixture pH was adjusted to 8.5 with 1 N hydrochloric acid solution. Dihydrazide adipate dissolved in 0.5 M sodium hydrogen carbonate was added to the mixture to be 0.3 M solution. The mixture was stirred at 4°C for one day. The mixture was centrifuged. The supernatant liquid was dialyzed with 0.2 M sodium chloride solution and gel filtrated with Bio Gelp-2 column (Bio-RedLaboratories, Richmond, U.S.A.). Fractions including saccharide and amino acid were collected, dialyzed with distilled water, and freeze-dried.

The obtained substance and bovine serum albumin (BSA) were dissolved in distilled water. After the mixture pH was adjusted to 4.9 with 0.2 N hydrochloric acid solution, 1-ethyl-3-(3-dimethylaminopropyl)carbodiimido was added to the mixture to be 0.1 M solution. The obtained mixture was stirred for three hours for bonding reaction. After the reacted solution was dialyzed with 0.2 M sodium chloride solution, the solution was centrifuged and the supernatant liquid was extracted. The reaction product was purified by gel filtration with SephacrylS-300 column. Saccharide peak fraction including saccharide and protein were collected, dialyzed with distilled water for three days, and freeze-dried. This was called BSA bonding purified polysaccharide antigen (designated "bacterial pellicle polysaccharide-BSA" thereafter)

An 18-week-old hen of White Leghorn was used in an immune experiment. Above-prepared 0.4 mg of acterial pellicle polysaccharide-BSA's of A.a. Y4 strain and P.g. 381 strain was inoculated. After the first inoculation, three more inoculations were conducted every two week. Yolk antibody was purified by Aurisio and Sheiokov method. In a similar manner as mentioned above, IgY antibody to Fusobacterium nucleatum (designated "F.n." thereafter), IgY antibody to Campylobacter rectus (designated "C.r." thereafter), IgY antibody to Bacteroides forsythus (designate "B.f." thereafter), and IgY antibody to Treponema denticola (designated "T.d." thereafter) were prepared. Other than the method mentioned above, various kinds of periodontal disease bacteria are cultured in an anaerobic environment and the various kinds of cultured bacteria are killed with formalin. The dead bacteria may be used as an antigen. After the antigen is inoculated into an egg-laying hen four times, the hen lays eggs having a high antibody titer. These eggs are collected and powdered. Thus, powder IgY antibody is obtained.

Commercial powder products including 6 % and 20 % of antibody (Taiyo Kagaku Company) were used for IgY antibody to S.m.

(Experiment for antibody effect)

After a buccal cavity was washed with three kinds of solutions: a 0.2 % ethanol solution (designated "ET" thereafter), a mixture of 0.2 % ethanol solution and 0.05 % oolong tea polyphenol solution (designated "EOT" thereafter), and a mixture of 0.2% ET, 0.05% OET, and 6% IgY antibody to S.m., the number of S.m. was counted. The results are summarized in Table 3. From Table 3, it is clear that the number of total chain coccus was decreased after gargling with OET+ET while the number of S.m. coccus was not decreased. However, when IgY antibody to S.m. was added, the number of S.m. was dramatically decreased. Thus, it was found that addition of antibody was quite effective to reduce the number of bacteria in a buccal cavity. The OET was polyphenol extracted from powder oolong tea (Sun-oolong, made by Suntory Ltd.) made in Fujian province, China suspended with 45 % (v/v) ethanol. The OET has enough Gtase inhibitory acitivity.

Table 3

| Change of number of S.m in a buccal cavity after gargling | | | |
|---|---|---|---|
| Gargle | Average score plaque | Total chain coccus number $\times 10^6$ (CFU / ml) | S.m. coccus number $\times 10^6$ (CFU / ml) |
| 0.2 %ET | $3.75 \pm 0.53$ | $132 \pm 198$ | $23 \pm 29$ |
| 0.05%OET & 0.2%ET | $2.98 \pm 0.62$ | $86 \pm 162$ | $25 \pm 37$ |
| 0.05%OET, 0.2%ET, & IgY | $2.44 \pm 0.31$ | $64 \pm 77$ | $10 \pm 16$ |

(Experiment for synergistic effects of antibody and propolis)

Synergistic antibacterial effects of antibody and propolis were found. The results are summarized in Table 4.

Table 4

| Synergistic effects of antibody and propolis | | | |
|---|---|---|---|
| Gargle | Average score plaque | Total chain coccus number x10$^6$ (CFU / ml) | S.m. coccus number x10$^6$ (CFU / ml) |
| IgY & 0.2%ET | 3.75 ± 0.53 | 132 ± 198 | 23 ± 29 |
| IgY, xylitol, & 0.2%ET | 2.57 ± 0.47 | 71 ± 86 | 21 ± 24 |
| IgY, propolis, & 0.2%ET | 1.84 ± 0.51 | 34 ± 24 | 5 ± 12 |

(Preparation of composite including antibody)

First 05. g of saccharide derivative (xylitol was used in the embodiment) was dissolved in 10 g of water. A powder antibody of 0.1 g was added to form an antibody solution. On the other hand, 27 g of shellac was dissolved in 56 g of ethanol to make a shellac ethanol-solution. The antibody solution was mixed with the shellac ethanol-solution, and 2 g of propolis and 3 g of perfume were added to the solution mixture to form a composite.

(Experiment for effects of composite applied to tooth)

After a molar in a lower jaw of a rat not having any inherent disease was infected by MT8148(C) of S.m., the rat was fed with food M2000 for 58 days (Table 5) and for 60 days (Table 6). Then average caries score on the molar was measured. The results of composites without propolis are summarized in Table 5 and those with propolis are summarized in Table 6. In Tables 5 and 6, "A" represents the composite with powder antibody including 20% of IgY, "B" represents the composite with powder antibody including 6% of IgY, and "C" represents the composite with powder without any IgY.

Table 5

| Average caries score without adding propolis Average caries score (±standard deviation) | | | | |
|---|---|---|---|---|
| | Groove | Cheek side | Neighboring side | Total |
| A | 40.1 ± 2.5 | 2.1 ± 0.3 | 0.5 ± 0.3 | 42.8 ± 2.8 |
| B | 64.1 ± 3.7 | 5.6 ± 0.4 | 1.4 ± 0.6 | 71.1 ± 4.1 |
| C | 90.4 ± 1.1 | 9.6 ± 1.1 | 4.9 ± 0.8 | 104.9 ± 1.9 |

From Table 5, it was found that average caries score at any portion of the tooth decreased while antibody-concentration of the composite increased (concentration order: C<B<A). Thus, it is understood that addition of antibody can prevent dental caries.

Table 6

| Average caries score with adding propolis Average caries score (±standard deviation) | | | | |
|---|---|---|---|---|
| | Groove | Cheek side | Neighboring side | Total |
| A | 12.2 ± 1.5 | 0.8 ± 0.3 | 0.2 ± 0.1 | 13.2 ± 1.8 |
| B | 20.3 ± 1.9 | 1.4 ± 0.4 | 0.9 ± 0.2 | 22.8 ± 2.3 |
| C | 32.8 ± 2.6 | 4.2 ± 0.6 | 2.3 ± 0.4 | 39.3 ± 3.6 |

Similarly as stated in Table 5, it was found that average caries score at any portion of the tooth decreased while antibody-concentration of the composite increased (concentration order: C<B<A). Thus, it is understood that addition of antibody can prevent dental caries. Further, if Tables 5 and 6 are compared, average caries score at the same portion with the same antibody-concentration in Table 6 is smaller than that in Table 5. Thus, adding both antibody and propolis can give the composite much stronger antibacterial effect than adding only antibody.

The composite including anti-periodontal-disease-bacteria antibody was examined. FIG. 1 shows change of relative numbers of bacteria on a tooth of serious periodontal patient infected by A.a. when two kinds of composite not including and including IgY antibody to A.a. were applied. (The former is designated "manicure including A.a. antibody", the latter is "manicure not including A.a. antibody" in FIG. 1). In FIG. 1, the vertical axis represents relative number of bacteria, the initial number of bacteria being unity (100%), and the horizontal axis represents time (week). Thus, chronological change of relative number of bacteria is illustrated. The relative number is the ratio of the current number and the initial number just before applying the composites. In FIG. 1, closed circles represent the manicure including A.a. antibody while closed squares represent the manicure not including A.a. antibody. From FIG.1, it is understood that the relative number is constantly around 0.9 after two weeks when the manicure not including A.a. antibody was applied. On the contrary, the relative number decreases around 0.6 after two weeks and it further decreases even lower than 0.5 after three weeks when the manicure including A.a. antibody was applied. Thus, it is clear that adding antibody can give the film much higher antibacterial property since adding A.a. antibody differentiates the relative numbers by about 0.4. The similar results to those for IgY antibody to A.a. were obtained for IgY antibody to P.g., IgY antibody to F.n., IgY antibody to C.r., IgY antibody to B.f., and IgY antibody to T.d.

**Example 7**

A powder of 27.0 g of dried transparent white shellac was dissolved in 56.0 g of 99.5 % absolute ethanol. To the solution, 20 mg of dipotassium glycyrrhetate was added and the mixture was stirred to be homogenized. To the mixture, 2.0 g of fish scale flake paste (25 wt% of fish scale flake kneaded with 75 wt% of 99.5 % absolute alcohol) was added and the mixture was stirred with a mixer. The tooth coating composite obtained through these processes had a light brownish tint and was a suspension of high flowability. When the suspension was applied by brush to a living tooth, it was dried out in three second to form a film. The film had a very similar tint and pearl gloss to those of the tooth enamel. The film had a high resistance to exfoliation and it was not exfoliated for several days with mastication of regular eating.

**Example 8**

The following materials were mixed in a similar manner to that of the example 7.

| Shellac | 27.0 g |
|---|---|
| Absolute ethanol | 56.0 g |
| Dipotassium glycyrrhetate | 20 mg |
| Amyl formate | 7.0 g |
| Lavender oil | 6.0 g |

Addition of amyl formate and lavender oil could give refreshing feeling.

**Example 9**

The following materials were mixed in a similar manner to that of the example 7.

| Shellac | 27.0 g |
|---|---|
| Absolute ethanol | 56.0 g |
| Dipotassium glycyrrhetate | 20 mg |

This composite is the most basic one that includes single efficacious constituent (dipotassium glycyrrhetate) dissolved in shellac alcohol solution.

**Example 10**

The following materials were mixed in a similar manner to that of the example 7.

| Shellac | 27.0 g |
|---|---|
| Absolute ethanol | 56.0 g |
| Dipotassium glycyrrhetate | 20 mg |
| Fish scale flake paste | 2.0 g |
| Peppermint | 4.0 g |
| Ethyl acetate | 7.0 g |

Addition of the fish scale flake paste could give the film a pearl gloss. Addition of the peppermint could supply freshness in a buccal cavity.

**Example 11**

The following materials were mixed in a similar manner to that of the example 7.

| Shellac | 27.0 g |
|---|---|
| Absolute ethanol | 56.0 g |
| Dipotassium glycyrrhetate | 20 mg |
| Fish scale flake paste | 2.0 g |
| Titanium oxide | 0.4 g |
| Peppermint | 4.0 g |
| Ethyl acetate | 6.5 g |

Addition of the titanium oxide could give the film a white color tone.

**Example 12**

The following materials were mixed in a similar manner to that of the example 7. In this example, shellac (emulsoid white shellac) was used to give a hardened film a white opaque color.

| Shellac (emulsoid white shellac) | 27.0 g |
|---|---|
| Absolute ethanol | 56.0 g |
| Dipotassium glycyrrhetate | 20 mg |
| Fish scale flake paste | 2.0 g |
| Titanium oxide | 0.4 g |
| Ethyl acetate | 6.5 g |

### Example 13

A powder of 27.0 g of dried transparent white shellac was dissolved in 56.0 g of 99.5 % absolute ethanol. To the solution, 4 mg of chlorhexidine gluconate was added and the mixture was stirred to be homogenized. To the mixture, 2.0 g of fish scale flake paste (25 wt% of fish scale flake kneaded with 75 wt% of 99.5 % absolute alcohol) was added and the mixture was stirred with a mixer. The tooth coating composite obtained through these processes had a light brownish tint and was a suspension of high flowability. When the suspension was applied by brush to a living tooth, it was dried out in three second to form a film. The film had a very similar tint and pearl gloss to those of the tooth enamel. The film had a high resistance to exfoliation and it was not exfoliated for several days with mastication of regular eating.

### Example 14

The following materials were mixed in a similar manner to that of the example 13.

| | |
|---|---|
| Shellac | 27.0 g |
| Absolute ethanol | 56.0 g |
| Chlorhexidine gluconate | 4 mg |
| Amyl formate | 7.0 g |
| Lavender oil | 6.0 g |

Addition of amyl formate and lavender oil could give refreshing feeling.

### Example 15

The following materials were mixed in a similar manner to that of the example 13.

| | |
|---|---|
| Shellac | 27.0 g |
| Absolute ethanol | 56.0 g |
| Chlorhexidine gluconate | 4 mg |

This composite is the most basic one that includes single anti-bacterial constituent (chlorhexidine gluconate) dissolved in shellac alcohol solution.

### Example 16

The following materials were mixed in a similar manner to that of the example 13.

| | |
|---|---|
| Shellac | 27.0 g |
| Absolute ethanol | 56.0 g |
| Chlorhexidine gluconate | 4 mg |
| Fish scale flake paste | 2.0 g |
| Peppermint | 4.0 g |
| Ethyl acetate | 7.0 g |

Addition of the fish scale flake paste could give the film a pearl gloss. Addition of the peppermint could supply freshness in a buccal cavity.

**Example 17**

The following materials were mixed in a similar manner to that of the example 13.

| Shellac | 27.0 g |
| Absolute ethanol | 56.0 g |
| Chlorhexidine gluconate | 4 mg |
| Fish scale flake paste | 2.0 g |
| Titanium oxide | 0.4 g |
| Peppermint | 4.0 g |
| Ethyl acetate | 6.5 g |

Addition of the titanium oxide could give the film a white color tone.

**Example 18**

The following materials were mixed in a similar manner to that of the example 13. In this example, shellac (emulsoid white shellac) was used to give a hardened film a white opaque color.

| Shellac (emulsoid white shellac) | 27.0 g |
| Absolute ethanol | 56.0 g |
| Chlorhexidine gluconate | 4 mg |
| Fish scale flake paste | 2.0 g |
| Titanium oxide | 0.4 g |
| Ethyl acetate | 6.5 g |

(Efficacious Test)

Now effects of efficacious constituent on periodontal disease was examined with a coating on a tooth formed from the composite of Example 9. The effects were measured by an improvement of periodontal disease index. The periodontal disease index is composed of PMA index, reddening index, swelling index, and plaque index. The PMA index is summation of points in P(reddening or swelling in papillar gingivae between teeth), M(reddening or swelling in marginal gingivae), and A(reddening or swelling around adherent gingivae), each of which has a point from 1 to 5 depending on how much inflamed gingivae is. The reddening index is to evaluate inflammation degree of gingivae around a tooth. Four faces around the tooth, i.e., front, back, cheek, and tongue faces are evaluated by the point of 0 (normal), 1 (subinflammatory, slight reddening, no hemorrhage), 2 (moderate inflammatory, reddening, hemorrhage on probing and pressing), and 3 (severe inflammatory, remarkable reddening, ulcer, hemorrhage). These points are added together and the total is divided by 4 to obtain the reddening index. The swelling index is to evaluate gingivae inflammatory degree. Four faces around the tooth, i.e., front, back, cheek, and tongue faces are evaluated by the point of 0 (normal), 1 (subinflammatory, no hemorrhage), 2 (moderate inflammatory, swelling, hemorrhage on probing and pressing), and 3 (severe inflammatory, remarkable swelling, ulcer, hemorrhage). These points are added together and the total is divided by 4 to obtain the reddening index. The plaque index is to evaluate plaque adhesion degree. Four faces around the tooth, i.e., front, back, cheek, and tongue faces are evaluated by the point of 0 (no plaque), 1 (plaque along gingivae margin), 2 (medium amount of plaque visible to the naked eye along gingivae margin), and 3 (heavy amount of plaque along gingivae margin and neighboring faces). These points are added together and the total is divided by 4 to obtain the plaque index. An efficacious effect of Example 9 composite was also evaluated in comparison with a blank composite (mixture of 27.0 g of shellac and 56.0 g of absolute ethanol). Two maxillary front teeth of 15 people were used. The composite of the present invention of Example 9 was applied to a flat surface of one tooth of each person. The blank composite was applied to a flat surface of the other. Four kinds of index numbers of each people were added together and each total was divided by 15 to obtain each arithmetic average. The initial index numbers were measured before the blank and Example 9 composites were applied to the teeth. Then, two composites were applied to cervicalis of the teeth (boundary between teeth and gingivae) everyday after brushing. Each index number was measured after one to four weeks. Four index numbers after one, two, three, and four weeks are added together and arithmetically averaged to obtain the index number after application. The results are summarized in Table 7.

Table 7

| Composite with dipotassium glycyrrhetate (periodontal disease index) | | | | | | | |
|---|---|---|---|---|---|---|---|
| Index | | Initial | 1 week | 2 weeks | 3 weeks | 4 weeks | after application |
| PMA index | Example 9 | 3.97 | 0.67 | 0.54 | 0.58 | 0.95 | 0.69 |
| | blank | 3.97 | 2.67 | 2.55 | 2.68 | 2.81 | 2.68 |
| Reddening index | Example 9 | 2.77 | 0.22 | 0.34 | 0.45 | 0.87 | 0.47 |
| | blank | 2.56 | 1.54 | 1.67 | 1.74 | 1.91 | 1.72 |
| Swelling index | Example 9 | 3.42 | 0.41 | 0.55 | 0.68 | 0.83 | 0.62 |
| | blank | 3.95 | 1.65 | 1.77 | 1.96 | 2.05 | 1.86 |
| Plaque index | Example 9 | 3.21 | 0.21 | 0.65 | 0.88 | 1.02 | 0.69 |
| | blank | 3.48 | 0.86 | 0.97 | 1.32 | 1.86 | 1.25 |

In Table 7, each of PMA index, reddening index, swelling index, and plaque index of Example 9 (composite of Example 9 was applied) are smaller than that of the blank to show positive effects of Example 9 composite against periodontal disease. The composite of the present invention can cure periodontal disease as well as prevent dental caries and periodontal disease. Since dipotassium glycyrrhetate has an anti-inflammatory function, applying the composite including it may enable to lower the periodontal index extensively (improve periodontal disease disorder).

(Anti-bacterial experiment 1)

Next, anti-bacterial effects were examined when the composite of Example 15 was applied to teeth. Two maxillary front teeth of 15 people were used. The composite of the present invention of Example 15 was applied to flat surface of one tooth of each person. A blank composite (mixture of 27.0 g of shellac and 56.0 g of absolute ethanol) was applied to flat surface of the other. Thus, both composites of Example 15 and the blank were applied to 15 teeth respectively. After two weeks of the application, DIs of both teeth to which the composite of Example 15 had been applied and to which the blank composite had been applied were measured. The term "DI" represents adhesion degree of dental plaque, which is evaluated by numbers of 0 (no dental plaque precipitate adhesion), 1 (dental plaque precipitate adhesion or adventitious coloring material on one-third of surface), and 2 (dental plaque precipitate adhesion or adventitious coloring material on one-third to two-thirds of surface), and 3 (dental plaque precipitate adhesion or adventitious coloring material on more than two-thirds of surface). All index numbers of each group are added together and the total is divided by 15 to obtain an arithmetic average. The results are summarized in Table 8.

Table 8

| Effects of chlorhexidine gluconate inclusion | | |
|---|---|---|
| Example 15 | DI | 1.024 |
| | standard deviation | 0.125 |

Table 8 (continued)

| Effects of chlorhexidine gluconate inclusion | | |
|---|---|---|
| Blank | DI | 1.531 |
| | standard deviation | 0.094 |

In Table 8, it is clear that applying the composite of Example 15 to the teeth could inhibit dental plaque adhesion by 33.1 %, which may be calculated by the formula:

$$(1.531\text{-}1.024)/1.531 \times 100 = 33.1\%$$

Therefore, the composite of Example 15, which includes chlorhexidine gluconate, is applied to a tooth to form coating (film) on it. Since the coating has an anti-bacteria function, the coating can effectively prevent dental plaque from adhering or sticking to the tooth surface.

(Anti-bacterial experiment)

After brushing the teeth by bass method, bacteria detecting film (TAC film) was applied to maxillary front teeth of 15 people. The composites of the present invention of Example 15 and a blank composite (mixture of 27.0 g of shellac and 56.0 g of absolute ethanol) were applied to different portions of the applied film. After two days, the bacteria detecting film (TAC film) was removed and each portion to which each composite had been applied was cultured. Then, the numbers of aerobic bacteria and anerobic bacteria were measured. The numbers of 15 people were arithmetically averaged. The results are summarized in Table 9.

Table 9

| Preventive effects on proliferation of dental plaque bacteria by inclusion of chlorhexidine gluconate | | |
|---|---|---|
| | | number of bacteria |
| aerobic | Example 15 | 200 |
| bacteria | Blank | 2000 |
| anerobic | Example 15 | 500 |
| bacteria | Blank | 1000 |

In Table 9, it is understood that the number of aerobic bacteria (chiefly dental caries bacteria) could be reduced to about one-tenth and that the number of anerobic bacteria (chiefly periodontal disease bacteria) could be reduced to about half by applying the composite of the present invention. Thus, coating on the tooth surface which has a anti-bacteria function against both aerobic and anerobic bacteria can be formed by applying the composite of the present invention which includes chlorhexidine gluconate.

The present invention, having the features mentioned above, can perform the following functions. The composite of the present invention comprising shellac dissolved in alcohol and at least one of antibacterial constituent, anti-bacteria antibody, and efficacious constituent is applied to a tooth surface to form an antibacterial film on the tooth surface such that it can prevent effectively dental caries and periodontal disease and cure periodontal disease. Further, it is possible to apply the composite to a tooth without any special technical skill such that it is quite easy to prevent dental caries and periodontal disease without any help of the dentist.

**Claims**

1. A tooth coating composite comprising shellac dissolved in alcohol wherein said composite has 1 to 50 wt% of shellac.

2. The tooth coating composite of claim 1 further comprising at least one of antibacterial constituent, anti-bacteria antibody, and efficacious constituent wherein said alcohol is ethanol.

3. The tooth coating composite of claim 2,

said anti-bacterial constituent comprising at least one of propolis, hinokitiol and chitin chitosan; and
said anti-bacteria antibody comprising anti-dental-caries-bacteria antibody and/or anti-periodontal-disease-bacteria antibody.

4. The tooth coating composite of claim 2 or 3,

said antibacterial constituent comprising disinfectant,
said disinfectant further comprising at least one of quaternary ammonium salt or quaternary ammonium salt-type disinfectant, fluoride or fluoride-type disinfectant, bisbiguanide derivative or bisbiguanide derivative-type disinfectant, phenol or phenol-type disinfectant, and amphoteric surface active agent or amphoteric surface active agent-type disinfectant.

5. The tooth coating composite of claim 2 to 4,

said anti-bacterial constituent comprising disinfectant;
said disinfectant further comprising at least one of quaternary ammonium salt or quaternary ammonium salt-type disinfectant, fluoride or fluoride-type disinfectant, bisbiguanide derivative or bisbiguanide derivative-type disinfectant, phenol or phenol-type disinfectant, and amphoteric surface active agent or amphoteric surface active agent-type disinfectant;
said quaternary ammonium salt or quaternary ammonium salt-type disinfectant further comprising at least one of cetylpyridinium chloride, benzalkonium chloride, benzethonium chloride, and dequalinium chloride;
said fluoride or fluoride-type disinfectant further comprising at least one of sodium fluoride, sodium monofluorophosphate, strontium fluoride,
said bisbiguanide derivative or bisbiguanide derivative-type disinfectant further comprising chlorhexidine;
said phenol or phenol-type disinfectant further comprising at least one of triclosan, thymol, and isopropyl methyl phenol; and
said amphoteric surface active agent or amphoteric surface active agent-type disinfectant further comprising alkyldiaminoethylglycine chloride.

6. The tooth coating composite of claim 2 to 5,

said anti-bacteria antibody comprising anti-dental-caries-bacteria antibody and/or anti-periodontal-disease-bacteria antibody;
said anti-dental-caries-bacteria antibody comprising IgY antibody to Streptococcus mutans; and
said anti-periodontal-disease-bacteria antibody comprising at least one of IgY antibody to Actinobacillus actinomycetemcomitans, IgY antibody to Porphyronomas gingivalis, IgY antibody to Fusobacterium nucleatum, IgY antibody to Campylobacter rectus, IgY antibody to Bacteroides forsythus, and IgY antibody to Treponema denticola.

7. The tooth coating composite of claim 2 to 6,

said efficacious constituent comprising at least one of tranexamic acid, allantoin, allantoindihydroxyaluminium, allantoinchlorohydroxyaluminium, $\varepsilon$-aminocaproic acid, lysozyme, sodium chloride, azulene, dl-$\alpha$-tocopherol acetate, dl-$\alpha$-tocopherol nicotinate, epidihydrocholesterol, dihydrocholesterol, Phellodendron Bark extract, zinc citrate, gantrosan, catechin, $\beta$-glycyrrhizinate, and glycyrrhetic acid.

8. The tooth coating composite of claim 2 to 7 further comprising at least one of brightener, pigment, and perfume.

9. The tooth coating composite of claim 8,

said brightener further comprising at least one of fish scale flake, titanated mica, sericite, muscovite, Pinctada shell flake, Abalone shell flake, and Rochina niloticus shell flake;
said pigment further comprising at least one of titanium oxide, titanated mica, natural pearl, yellow oxide of iron, calcium (II) phosphate, calcium carbonate, and hydroxyapatite; and
said perfume further comprising at least one of ethyl acetate, amyl formate, lavender oil, rosemary oil, peppermint, methyl butyrate, isoamyl isovalerate, benzyl acetate, and isoamyl acetate.

10. A method for preparing the tooth coating composite of claim 2 to 9, wherein said anti-bacteria antibody comprises anti-dental-canes-bacteria antibody and/or anti-periodontal-disease-bacteria, comprising steps of:

      making a mixture of water, saccharide derivative, and said anti-dental-caries-bacteria antibody and/or said anti-periodontal-disease bacteria antibody; and
      mixing said mixture with alcohol solution including shellac.

Change of relative number of bacteria after antibacterial manicure was applied to a tooth of serious periodontal disease patient infected by A.a.

## A.a.

relative number of bacteria

Relative numbers are shown in a ratio to the initial number of the bacteria (at 0 week).

● A.a.  Manicure including A.a. antibody

■ A.a.  Manicure not including A.a. antibody

European Patent Office

# EUROPEAN SEARCH REPORT

**Application Number**

| | DOCUMENTS CONSIDERED TO BE RELEVANT | | EP 98117005.3 |
|---|---|---|---|
| Category | Citation of document with indication, where appropriate, of relevant passages | Relevant to claim | CLASSIFICATION OF THE APPLICATION (Int. Cl. 6) |
| X | Patent Abstracts of Japan, Vol. 16, No. 298 (C-958), 1992; & JP 04-082821 A (JUN KAWAI). -- | 1,2, 8,9 | A 61 K 7/16 C 09 D 193/02 |
| A | DE 1965046 A1 (BLENDAX-WERKE R.SCHNEIDER & CO.) 01 July 1971 (01.07.71), claims 1-4. -- | 1,4,5 | |
| A | AT 172063 B (A. JENKÓ) 11 August 1952 (11.08.52), totality. ---- | 2,3 | |
| | | | **TECHNICAL FIELDS SEARCHED (Int. Cl. 6)** |
| | | | A 61 K 7/00 C 09 D 193/00 |

The present search report has been drawn up for all claims

| Place of search | Date of completion of the search | Examiner |
|---|---|---|
| VIENNA | 26-11-1998 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04011)